# EUROPÄISCHE PATENTSCHRIFT

(11) **EP 2 683 448 B1**
(45) Veröffentlichungstag und Bekanntmachung des Hinweises auf die Patenterteilung: **14.10.2020**
(21) Anmeldenummer: 12708323.6
(22) Anmeldetag: 09.03.2012
(51) Int. Cl.: A61K 8/64, A61K 8/04, A61K 8/896, A61K 8/92, A61Q 9/02, A61Q 19/00, A61Q 19/10, A23P 30/40, A23L 27/00, A61K 8/73

(54) **WIRKSTOFFHALTIGER SCHAUM ENTHALTEND EIN PROTEIN UND EINEN FETTSÄUREESTER SOWIE VERFAHREN ZU DESSEN HERSTELLUNG**
FOAM CONTAINING AN ACTIVE INGREDIENT, A PROTEIN AND A FATTY ACID ESTER AND METHOD FOR ITS PREPARATION
MOUSSE CONTENANT UN AGENT ACTIF, UN PROTÉIN ET UN ESTER D'UN ACIDE GRAS ET PROCÉDÉ DE PRÉPARATION CORRESPONDANTE

(30) Priorität: 09.03.2011 DE 102011013447; 06.07.2011 DE 102011106697
(43) Veröffentlichungstag der Anmeldung: 15.01.2014
(73) Patentinhaber: Fraunhofer-Gesellschaft zur Förderung der angewandten Forschung e.V., 80686 München (DE)
(72) Erfinder: BRUZZANO, Stefano, 47051 Duisburg (DE); PFEIFER, Sebastian, 47055 Duisburg (DE); SOMBORN-SCHULZ, Annette, 45661 Recklinghausen (DE); BRETZ, Karlheinz, 46049 Oberhausen (DE)
(74) Vertreter: Weishäupl, Michael
(86) Internationale Anmeldenummer: PCT/EP2012/054159
(87) Internationale Veröffentlichungsnummer: WO 2012/120134

(56) Entgegenhaltungen:
- EP-A1- 1 129 699
- CH-A5- 674 804
- DE-A1- 10 159 002
- GB-A- 1 170 092
- GB-A- 2 242 198
- GARTI ET AL: "WHAT CAN NATURE OFFER FROM AN EMULSIFIER POINT OF VIEW:TRENDS AND PROGRESS?", COLLOIDS AND SURFACES A: PHYSIOCHEMICAL AND ENGINEERING ASPECTS, ELSEVIER, AMSTERDAM, NL, vol. 152, no. 1-2, 1 January 1999 (1999-01-01), pages 125-146, XP008079746, ISSN: 0927-7757, DOI: 10.1016/S0927-7757(98)00621-9
- DATABASE GNPD [Online] MINTEL; 7 February 2007 (2007-02-07), anonymous: "Soymilk Smoothie", XP055644337, retrieved from www.gnpd.com Database accession no. 654445

## Beschreibung

Die Erfindung betrifft ein Verfahren zur Herstellung eines wirkstoffhaltigen Schaums, der zum einen eine Fettsäureesterkomponente, also ein Fett und/oder ein Wachs, und zum anderen ein Protein umfasst. Die Ausgangsmaterialien sind dabei im Wesentlichen biogenen Ursprungs und stellen in Form eines wässrigen Gemischs eine aufschäumbare Zusammensetzung dar. Zur Herstellung eines Schaums wird in diese Zusammensetzung ein Gas eingebracht.

Nach dem Stand der Technik werden in der Kosmetik vielseitig Schäume als weiche Formulierungen eingesetzt (z. B. als Rasier-, Gesichts-, Dusch- und Fußpflegeschäume), da sie eine sehr gute Haptik aufweisen, sich also bei der Anwendung angenehm auf der Haut anfühlen.

Problematisch ist allerdings, dass in den meisten Schäumen Verbindungen wie Natriumlaurylsulfat oder Polyethylenglykol-Glycerylcocoate, also synthetische Tenside, enthalten sind. Diese Zusätze werden auf Grund ihrer schaumbildenden bzw. emulgierenden Eigenschaften zugesetzt. Sie haben aber den Nachteil, dass sie oft zu unerwünschten Hautreaktionen oder anderen Unverträglichkeiten führen. Sie gelten ferner als Promotoren für Kontaktallergien, da sie bei regelmäßigem Gebrauch die Haut austrocknen und den Fett- und Säureschutzmantel angreifen. Dadurch können sie die Haut auch auf andere Inhaltsstoffe sensibilisieren.

Um die genannten Tenside zu ersetzen, wurden Pickering-Emulsionen vorgeschlagen, bei denen eine Stabilisierung durch Feststoffnanopartikel erfolgt, die sowohl mit hydrophilen als auch mit lipophilen Flüssigkeiten benetzbar sein müssen. Hinsichtlich medizinischer und kosmetischer Anwendung wird die Verwendung von Nanopartikeln allerdings kontrovers diskutiert, da die gesundheitliche Unbedenklichkeit, insbesondere bzgl. der Langzeitfolgen, noch nicht abgeschätzt werden kann.

Die GB 1 170 092 A offenbart eine schaumförmige Zusammensetzung, bei der eine Mischung, die ein pflanzliches Öl, Leberextrakt, einen Schaumstabilisator wie Nipagin und einen Wirkstoff umfasst, emulgiert und dann mit Frigen aufgeschäumt wird. Die GB 2 242 198 A bezieht sich auf kosmetische Zusammensetzungen. Hierbei wird ein Gemisch, umfassend ein pflanzliches Öl, Seidenpeptid, den Schaumstabilisator Dimethylpolysiloxan und einen Wirkstoff, mit LP-Gas in einem Druckbehälter gemischt. Gemäß der CH 674 804 A5 wird eine kosmetische Creme erhalten, die auch als Schaum vorliegen kann. Dieser Schaum wird erhalten durch Unterheben von Eischnee in eine Mischung, die eine Fettsäureesterkomponente, Kollagen, den Schaumstabilisator Hydrorhamnoson und einen Wirkstoff umfasst. In allen drei vorstehend genannten Entgegenhaltungen wird die Schaumstabilisierung unter anderem durch Zugabe eines synthetischen Tensids erreicht.

Eine Aufgabe der Erfindung ist es, einen wirkstoffhaltigen Schaum und ein Verfahren zu dessen Herstellung anzugeben, der verträglicher ist für Körpergewebe, insbesondere für Haut, als die Schäume nach dem Stand der Technik.

Diese Aufgabe wird durch das Verfahren zur Herstellung eines wirkstoffhaltigen Schaums gemäß dem Hauptanspruch und den wirkstoffhaltigen Schaum gemäß den unabhängigen Ansprüchen gelöst. Weitere Ausgestaltungen und Weiterbildungen sind Gegenstand von Unteransprüchen und gehen weiterhin aus der nachfolgenden Beschreibung hervor.

Ein Verfahren zur Herstellung eines wirkstoffhaltigen Schaums, der zum einen ein Protein und zum anderen eine Fettsäureesterkomponente (also ein Fett und/oder ein Wachs) umfasst, weist die folgenden Schritte auf:
A) Zunächst wird die Fettsäureester und die Proteinkomponente bereit gestellt; die Fettsäureesterkomponente und/oder die Proteinkomponente werden dabei so ausgewählt, dass sie einen schaumstabilisierenden Bestandteil enthalten, wobei entweder die Proteinkomponente ein schaumstabilisierendes Protein enthält oder die Fettsäureesterkomponente einen schaumstabilisierenden Bestandteil enthält oder sowohl die Proteinkomponente als auch die Fettsäureesterkomponente einen schaumstabilisierenden Bestandteil enthält, nämlich ein schaumstabilisierendes Protein bzw. einen schaumstabilisierenden Fettsäureester-Bestandteil. Alternativ kann die Proteinkomponente aus dem schaumstabilisierenden Protein und/oder die Fettsäureesterkomponente aus dem schaumstabilisierenden Fettsäureester-Bestandteil bestehen. In die Fettsäureesterkomponente wird ferner die Wirkstoffkomponente eingebracht oder ist bereits eingebracht; wenn die Wirkstoffkomponente gasförmig ist, so kann die Wirkstoffkomponente im Einzelfall zumindest teilweise auch erst in Schritt C) eingebracht werden. Die Wirkstoffkomponente kann entweder ein pharmazeutischer und/oder ein kosmetischer Wirkstoff sein. Ferner werden die Fettsäureesterkomponente und die Proteinkomponente so ausgewählt, dass das Gewichtsverhältnis Fettsäureesterkomponente : Proteinkomponente von 1 : 10 bis 10 : 1 betragen kann (das Gewichtsverhältnis bezieht sich hierbei auf die Fettsäureesterkomponente ohne darin eingebrachten Wirkstoff); bei der Proteinkomponente ist ferner zu beachten, dass - wie generell im Rahmen dieser Anmeldung gilt - für die Bestimmungen des Anteils in Gewichtsprozent die wässrigen Bestandteile, die ggf. in einer Proteinkomponente enthalten sein können, nicht in den Anteil der Proteinkomponente mit eingerechnet sind. Wesentlich bei der Auswahl der Fettsäureesterkomponente und der Proteinkomponente ist, dass zumindest die Fettsäureesterkomponente, und gegebenenfalls auch die Proteinkomponente und der schaumstabilisierende Bestandteil im Wesentlichen biogenen Ursprungs sind.
B) In einem nachfolgenden Schritt werden die Proteinkomponente und die Fettsäureesterkomponente gemischt, wobei ein Verdicker und gegebenenfalls Wasser zugesetzt wird (insbesondere, wenn die Proteinkomponente nicht als wässriges Gemisch eingesetzt wird oder zu wenig Wasser bezüglich der Wassermenge, die für die herzustellende Zusammensetzung erforderlich ist, vorhanden ist, wobei keine synthetischen oder teilsynthetischen Tenside zugesetzt werden. Das Wasser einerseits und die aufsummierten Anteile von Proteinkomponente und Fettsäureesterkomponente andererseits liegen in einem Gewichtsverhältnis von 1 : 1 bis 20 : 1 vor (das Wasser liegt also im Regelfall im Überschuss vor). Das dabei erhaltene Gemisch weist einen Anteil der Fettsäureesterkomponente auf, der größer als 3 Gew.-% ist (bezogen auf die summierten Anteile von Wasser, Fettsäureesterkomponente, Proteinkomponente und Wirkstoff).
C) In das in Schritt B) erhaltene Gemisch wird schließlich ein fettlösliches Gas eingebracht, so dass ein Schaum entsteht.

Mit dem erfindungsgemäßen Verfahren kann ein wirkstoffhaltiger Schaum hergestellt werden, der gut dosierbar ist, was insbesondere aus dem hohen Gasvolumen im Verhältnis zum nicht gasförmigen Volumen (nachfolgend auch "Flüssigkeitsvolumen" bezeichnet) resultiert. Die Wirkstoffe lassen sich also gezielt niedrig dosiert und homogen verteilen. Im Unterschied zum Stand der Technik dienen erfindungsgemäß die Fettsäureesterkomponente und/oder die Proteinkomponente zur Schaumstabilisierung, so dass auf synthetische Tenside gänzlich verzichtet werden kann. Ferner kann bei der regelmäßigen Anwendung von Schäumen der Sensibilisierung der Haut vorgebeugt werden. Auch lassen sich Patienten mit Unverträglichkeiten gegen klassische Tenside wie Natriumlaurylsulfat oder auch gegen Glucotenside mit Hilfe der erfindungsgemäßen Schäume behandeln.

Auf Grund der Verwendung der Fettsäureesterkomponente bzw. der Proteinkomponente treten - verglichen mit den tensidhaltigen Schäumen nach dem Stand der Technik (den sogenannten Trockenschäumen) - gänzlich neue Eigenschaften, insbesondere eine völlig neue Haptik auf. Die erfindungsgemäßen Schäume fühlen sich feucht an, glänzen und bewirken auf Grund des relativ hohen Fettsäureesteranteils (also eines ölartigen Anteils) eine Rückfettung der Haut, so dass im Vergleich zu den Schäumen nach dem Stand der Technik keine Austrocknung erfolgt.

Ferner ist es auf Grund des relativ großen Ölanteils im wirkstoffhaltigen Schaum sehr leicht möglich, die Wirkstoffe ins Körpergewebe, insbesondere in die Haut, einzubringen. Die Fettsäureesterkomponente dient als Träger- und/oder Transportsystem für die Wirkstoffe, insbesondere für lipophile Wirkstoffe.

Im Vergleich zu reinen Ölen weisen die erfindungsgemäßen Schäume aber den Vorteil auf, dass sie wesentlich besser handhabbar bei der Applikation (z. B. beim Einreiben) sind, was nicht nur auf der besseren Dosierbarkeit beruht, sondern auch darauf, dass kein oder nur ein geringes Tropfen der aufgebrachten Masse erfolgt.

Schließlich ist noch darauf hinzuweisen, dass die erfindungsgemäßen wirkstoffhaltigen Schäume im Regelfall auch eine Komponentenzahl aufweisen, die gegenüber der herkömmlicher Formulierungen auf ein Minimum reduziert wurde, so dass generell ein geringeres Risiko besteht, durch einen der Inhaltsstoffe gesundheitlich beeinträchtigt zu werden.

Gemäß einer Ausführungsform bildet das in Schritt B) erhaltene Gemisch eine Emulsion aus. Insbesondere liegt dabei die zumindest die Fettsäureesterkomponente im Wasser als disperse Phase vor, insbesondere in Form von Tröpfchen. Prinzipiell ist aber auch denkbar, dass Wasser die disperse Phase ist. Der mittlere Teilchendurchmesser der dispersen Phase beträgt im Regelfall 20 nm bis 50 µm (gemessen mittels dynamischer Lichtstreuung), insbesondere 100 nm bis 10 µm. Es kann sich dabei beispielsweise um eine Emulsion mit einem mittleren Teilchendurchmesser größer 200nm (vergleichbar mit einer Makroemulsion), eine Emulsion mit einem mittleren Teilchendurchmesser von 100 nm bis 200nm (vergleichbar mit einer Miniemulsion) oder um eine transparente Emulsion mit einem mittleren Teilchendurchmesser von kleiner 100 nm (vergleichbar mit einer Mikroemulsion) handeln. Wie bereits vorstehend ausgeführt wurde, enthält die nach Schritt B) erhaltene Zusammensetzung keine synthetischen Tenside. Liegt diese nun als Emulsion vor, so bedeutet dies auch, dass diese Emulsion auch keine synthetischen Emulgatoren enthält.

Die erfindungsgemäß bereitgestellte Fettsäureesterkomponente, die Proteinkomponente und der schaumstabilisierende Bestandteil sind im Wesentlichen biogenen Ursprungs, wobei die Fettsäureesterkomponente aus einem Pflanzenöl oder einem pflanzlichen Wachs besteht, und wobei die Proteinkomponente ein pflanzliches Protein, ein Eierprotein oder ein Milchprotein umfasst oder daraus besteht.

Unter "biogenem Ursprung" ist "biologischen oder organischen Ursprungs" zu verstehen, der Begriff umfasst also nicht Materialien chemisch-synthetischer Herkunft. Der Begriff "Fettsäureesterkomponente, Proteinkomponente und schaumstabilisierender Bestandteil biogenen Ursprungs" umfasst somit alle die Fettsäureesterkomponenten, Proteinkomponenten und schaumstabilisierende Bestandteile, die von Pflanzen, Tieren oder Mikroorganismen (insbesondere Pilzen, Algen und Bakterien) gebildet werden.

"Im Wesentlichen" biogenen Ursprungs bedeutet dabei, dass zumindest 90 Gew.-% der Fettsäureesterkomponente, meist mehr als 95 Gew.-% und häufig mehr als 99 Gew.-% biogenen Ursprungs sind. Idealerweise sind 100 Gew.-% der Fettsäureesterkomponente 100 % biogenen Ursprungs.

Es gilt hierbei im Regelfall, dass sowohl 90 Gew.-% der Fettsäureesterkomponente als auch 90 Gew.-% der Proteinkomponente biogenen Ursprungs sind. Üblicherweise ist der Anteil beider Komponenten aber zu mehr als 95 % biogenen Ursprungs, häufig zu mehr als 99 Gew.-% biogenen Ursprungs. Idealerweise sind sowohl die Fettsäureesterkomponente als auch die Proteinkomponente zu 100 % biogenen Ursprungs. Entsprechend gilt für den schaumstabilisierenden Bestandteil, der zumindest teilweise in der Fettsäureesterkomponente und/oder der Proteinkomponente enthalten ist, dass dieser - unabhängig von den vorstehend angegebenen Zahlen für Fettsäureesterkomponente und Proteinkomponente - zumindest zu 90 % biogenen Ursprungs ist, im Regelfall zumindest zu 99 % biogenen Ursprungs ist und häufig zu 100 % biogenen Ursprungs ist.

Unter biogenem Ursprung kann darüber hinaus gemäß einer Ausführungsform auch verstanden werden, dass synthetisch hergestellte Materialien wie beispielsweise synthetisch hergestellte Triglyceride aus den natürlich vorkommenden Ölen enthaltenen Fettsäuren und unverseifbare Bestandteile von natürlichen Ölen (beispielsweise Squalen), die synthetisch hergestellt sind, so miteinander kombiniert werden, dass eine Zusammensetzung entsteht, die der erfindungsgemäß beschriebenen Materialien, die im Wesentlichen biogenen Ursprungs sind, entsprechen.

Gemäß einer Ausführungsform werden als im Wesentlichen biogene Materialien Materialien zugesetzt, die für die Lebensmittelindustrie zugelassen sind. Dies gilt unabhängig voneinander für die Fettsäureesterkomponente und die Proteinkomponente, bevorzugt gilt es für beide Komponenten. Beide Komponenten können unabhängig voneinander pflanzlichen und/oder tierischen Ursprungs sein.

Die erfindungsgemäß eingesetzte Fettsäureesterkomponente kann ein Fett sein, also ein Material, das im Wesentlichen aus Mono-, Di- und Triglyceriden besteht, wobei der Anteil von Mono- und Diglyceriden gegenüber den von Triglyceriden meist sehr gering ist (also meist < 5 Gew.-% der Gesamtglyceridmenge). Neben den Glyceriden sind im Regelfall noch unverseifbare Bestandteile in den Fetten enthalten, deren Anteil meist < 5 Gew.-% in der Fettsäureesterkomponente beträgt, aber im Einzelfall auch deutlich darüber liegen kann, wenn "exotischere" Öle für die Fettsäureesterkomponente eingesetzt werden, beispielsweise Jojoba-Öl.

Die erfindungsgemäß eingesetzte Fettsäureesterkomponente besteht aus Pflanzenölen oder Wachsen . Das Pflanzenöl kann dabei ausgewählt sein aus einem oder mehreren der nachfolgend beispielhaft genannten Öle: Amaranthöl, Aprikosenkernöl, Arganöl, Avellanaöl, Avocadoöl, Babassuöl, Baobaböl, Borretschsamenöl, Broccolisamenöl, Cupuaçuöl, Distelöl, Granatapfelsamenöl, Hanföl, Erdnussöl, Haselnussöl, Holundersamenöl, Jatrophaöl, Johannisbeersamenöl, Kakaobutter, Kameliensamenöl, Kokosöl, Kürbiskernöl, Macadamianussöl, Mandelöl, Maiskeimöl, Marulaöl, Nachtkerzen Öl, Olivenöl, Preiselbeersaamenöl, Reiskeimöl, Rapsöl, Reisöl, Sanddornöl, Schwarzkümmelöl, Sesamöl, Sheabutter, Sheanussöl, Sojaöl, Sonnenblumenöl, Traubenkernöl, Palmöl, Palmkernöl, Walnusskernöl, Weizenkeimöl, Wiesenschaumkrautöl und Wildrosenöl. Generell kann also ein beliebiges aus Pflanzen herstellbares Öl ausgewählt werden, wobei dann häufig als Bestandteile der Triglyceride Ölsäure und Palmitinsäure enthalten sind und häufig auch noch zusätzlich Linolsäure und/oder Stearinsäure.

Als geeignet haben sich insbesondere Öle erwiesen, die Lecithin enthalten, insbesondere in einem Anteil von zumindest 0,25 Gew.-%, insbesondere von 0,5 Gew.-%, zu nennen sind beispielsweise Sonnenblumenöl, Sojaöl, Weizenkeimöl oder Sesamöl. Ferner können als Öle auch solche Öle eingesetzt werden, die zumindest einen der folgenden Bestandteile enthalten: Vitamine, Mineralstoffe, Karotinoide, Phytosterole und Flavonoide, da diese vorteilhafte Auswirkungen, beispielsweise bei topischer Anwendung der erfindungsgemäßen Schäume besitzen können.

Bei der Auswahl der Öle ist gegebenenfalls auf die Stabilität, insbesondere Oxidationsstabilität zu achten. Bestimmte Öle (z.B. Leinöl) werden bei Luftkontakt relativ schnell ranzig, so dass bei Verwendung derartiger Öle die aufschäumbare Zusammensetzung unter Luftausschluss und möglichst auch unter Lichtausschluss z.B. in einer Druckgasdose gelagert werden sollte und erst direkt vor der Applikation aufgeschäumt werden sollte.

Neben Fetten bzw. Ölen kommen als Fettsäureesterkomponente insbesondere auch Wachse in Betracht. Hierbei handelt es sich um Materialien, die auf Estern von Fettsäuren basieren, wobei insbesondere einwertige Alkohole und nicht Glycerine als Alkoholkomponente fungieren. Der Anteil der Fettsäureesterkomponente beträgt üblicherweise zumindest 70 %; daneben können beispielsweise Paraffinkohlenwasserstoffe und andere unverseifbare Komponenten vorliegen. Als Wachse können beispielsweise Bienenwachs, Beerenwachs, Japanwachs, Jojobaöl, Wollwachs, Carnaubawachs und Zuckerrohrwachs genannt werden. Es können selbstverständlich auch Gemische dieser Wachse verwendet werden. Generell können also Wachse pflanzlicher und/oder tierischer Herkunft eingesetzt werden. Selbstverständlich stellen auch Gemische einer oder mehrerer Fette und einer oder mehrerer Wachse bereit gestellt werden.

Gemäß meiner Ausführungsform der Erfindung wird als Fettsäureesterkomponente ein Öl oder ein Wachs bereit gestellt, dass bei 37° Celsius flüssig ist. Dies hat den Vorteil, dass die Fettsäureesterkomponente beim Auftragen auf Gewebe, insbesondere Haut, in flüssigem Zustand vorliegen sollte und dementsprechend ein gutes Einziehen der Fettsäureesterkomponente und der darin enthaltenen Wirkstoffe möglich ist.

Gemäß einer weiteren Ausführungsform der Erfindung beträgt der Gewichtsanteil der Fettsäureesterkomponente des in Schritt B) erhaltenen Gemischs 5 bis 50 Gew.-%, insbesondere 10 bis 35 Gew.-%. Beispielsweise kann der Gewichtsanteil der Fettsäureesterkomponente 15 bis 20 Gew.-% betragen. Der Gewichtsanteil der Fettsäureesterkomponente ist also relativ hoch, was erfindungsgemäß erwünscht ist, um eine Rückfettung zu bewirken. Ferner kann bei einem hohen Fettsäureesteranteil auch eine große Menge lipophiler Wirkstoffe eingebracht werden.

Die erfindungsgemäße Proteinkomponente kann insbesondere ein Protein und/oder ein Proteinextrakt umfassen. Im Einzelfall kann als Proteinkomponente auch ein reines Protein bzw. ein Gemisch von mehreren der genannten Materialien eingesetzt werden; die Proteinkomponente besteht dann aus einem Protein und/oder einem Proteinextrakt. Da allerdings vorzugsweise biogene Materialien eingesetzt werden, enthält die Proteinkomponente neben dem Protein bzw. dem Proteinextrakt in den allermeisten Fällen auch noch weitere Stoffe. Der Proteingehalt in der Proteinkomponente beträgt üblicherweise mehr als 30 Gew.-% und in der Regel auch mehr als 50 Gew.-%. Häufig ist der Proteingehalt allerdings noch höher, insbesondere > 70 Gew.-%. Beispielsweise können die Proteingehalte auch durch Entölen und ähnliches stärker erhöht werden; insbesondere in diesen Fällen können auch Proteingehalte > 80 Gew.-% und sogar > 90 Gew.-% erreicht werden. Der Proteingehalt ist dabei definiert als der Gehalt, der sich aus der Bestimmung des Stickstoffs und dessen Multiplikation mit dem Faktor 6,25 errechnet. Der Stickstoffgehalt wird dabei mittels herkömmlicher Elementaranalyse ermittelt. Der Anteil der Proteinkomponente in dem Gemisch, das nach Schritt B) erhalten wird beträgt üblicherweise zwischen 5 und 25 Gew.-%, insbesondere zwischen 10 und 20 Gew.-%.

Die anmeldungsgemäß eingesetzten Proteine bzw. Proteinextrakte können sowohl pflanzlichen als auch tierischen Ursprungs sein. Als tierische Proteine werden anmeldungsgemäß Ei-, beispielsweise Hühnerei-Proteine, sowie Milch-Proteine und deren Extrakte, die beispielsweise in Form von Hydrolysaten vorliegen können, eingesetzt. Häufig weisen die tierischen Proteinpräparate allerdings einen charakteristischen Eigengeschmack und Eigengeruch auf, daher sind pflanzliche Proteine und von den tierischen Proteinen die Milch-Proteine tendenziell eher bevorzugt. Als pflanzliche Proteine sind insbesondere Getreide-Proteine (z. B. Weizenkleber-Präparate), Hülsenfrucht-Proteine (z. B. Soja-Proteine, Erbsen-Proteine oder Lupinen-Proteine) oder auch Proteine aus Samen (z. B. Rapssamen oder Sonnenblumenkerne) sowie Proteine aus Knollenpflanzen (z. B. Kartoffel-Proteine) zu nennen.

Zur Proteinextraktion können beispielsweise die pflanzlichen oder tierischen Ausgangsstoffe zunächst entölt werden (etwa mit lipophilen Lösungsmitteln). Auch eine alternative oder zusätzliche Flockierung oder ein Pressen der pflanzlichen und tierischen Ausgangsmaterialien ist denkbar. Durch die Extraktion kann beispielsweise der Restölgehalt in der Proteinkomponente auf ≤ 5 Gew.-% reduziert werden. Ferner kann eine Proteinextraktion mittels Walzentrocknung erfolgen.

Als Proteinkomponente können aber auch bereits aus den Naturstoffen isolierte Proteine eingesetzt werden. Hier sind beispielsweise zu nennen Albumine (tierisch), Globulin (tierisch), Glutelin (Getreide), Histone, Protamine (tierisch), konjugierte Proteine wie Phosphoproteine, Chromoproteine, Lecithoproteine, Peptone. Erfindungsgemäß können aber auch Proteinkomponenten eingesetzt werden, die diese Proteine neben anderen Stoffen enthalten.

Wie ausgeführt, kann die Proteinkomponente einen Restfettgehalt aufweisen. Im Regelfall liegt dieser Restfettgehalt allerdings unter 10 Gew.-%, so dass beim Fettgehalt des gesamten Schaums der Fettgehalt des Proteins gegenüber dem Gehalte der Fettsäureester-Komponente eine eher untergeordnete Rolle spielt. Häufig ist der Restfettgehalt der Proteinkomponente sogar geringer als 5 Gew.-%. Am fertigen wirkstoffhaltigen Schaum kann dies auch daran erkannt werden, dass die im Gesamtgemisch enthaltenen Fettsäuren im Wesentlichen denen der Fettsäureesterkomponente entsprechen. Zudem wird häufig die Fettsäureesterkomponente und die Proteinkomponente nicht denselben pflanzlichen bzw. tierischen Ursprung haben, sondern aus unterschiedlichen Pflanzenarten bzw. Tieren gewonnen sein. Insbesondere sind die in Schritt A) bereitsgestellte Fettsäureesterkomponente und Proteinkomponente nicht zueinander identisch und ergeben auch nach Schritt B) keine Zusammensetzung, die als solche nicht unter dem Begriff eines "biogenen" Ausgangsmaterials gemäß der vorliegenden Anmeldung, wie es in Schritt A) für die Proteinkomponente oder die Fettsäureesterkomponente verwendet werden kann, subsummiert werden kann. Wird beispielsweise ein pflanzliches Öl als Fettsäureesterkomponente verwendet und ein Milchprotein oder ein Trockenmilchpulver als Proteinkomponente, so ist ein Fettgehalt des wirkstoffhaltigen Schaums der Anteil, der auf die Milch-Komponente zurückgeht im Regelfall schon daran festzumachen, dass Milch einen sehr viel höheren Anteil an kurzkettigen "Fettsäuren" mit vier bis fünfzehn Kohlenstoffatomen enthält, der bis zu 35 Gew.-% im Milchfett betragen kann.

Als Proteinkomponente kann schließlich auch neben den beispielsweise als Hydrolysat vorliegenden Proteinextrakten der Einsatz modifizierter Proteine erfolgen. Die in der Proteinkomponente enthaltenen Proteine können also auch - müssen aber nicht - chemisch modifiziert sein, insbesondere acetyliert, verestert, desaminiert und/oder amidiert. Die chemische Modifikation kann beispielsweise biochemisch, insbesondere enzymatisch, aber grundsätzlich auch mittels organisch/anorganisch-chemischer Reagenzien erfolgen. Diese modifizierten Proteine sind gemäß einer Ausführungsform dieser Anmeldung, zumindest, wenn es sich um biochemische Modifikationen eines Proteins handelt, ebenfalls als biogenen Ursprungs anzusehen, wenn das Proteingrundgerüst nicht synthetisch aufgebaut wurde sondern aus einem biogenen Material erhalten wurde, da lediglich im geringen Ausmaß eine chemische Modifikation der vorhandenen funktionellen Gruppen des Proteins erfolgt.

Gemäß einer Ausführungsform weisen zumindest 75 Gew.-% der in der Proteinkomponente enthaltenen Proteine ein Molekulargewicht ≤ 100 kDa, insbesondere ≤ 10 kDa, auf, im Regelfall werden sogar mehr als 90 Gew.-% der in der Proteinkomponente enthaltenen Proteine unter einer oder beiden der genannten Schranken liegen und häufig auch mehr als 95 Gew.-% unter einer oder beiden der genannten Schranken. Eine derartige Reduzierung auf eher kürzerkettige Proteine hat den Vorteil, dass bei Anwendungen, die die Applikation des wirkstoffhaltigen Schaums auf der Haut vorsehen, kein Abrieb entstehen sollte, der sich bei größeren Molekülmassen bilden würde und optisch ein unerwünschtes Erscheinungsbild des applizierten Schaums hinterlässt.

Der wesentlichste Bestandteil der erfindungsgemäßen wirkstoffhaltigen Schäume ist der schaumstabilisierende Bestandteil. Unter einem schaumstabilisierenden Bestandteil wird dabei verstanden, dass in der Schaumzusammensetzung Moleküle oder Molekülteile enthalten sind, die die Bildung von Gasblasen stabilisieren. Der schaumstabilisierende Bestandteil ist erfindungsgemäß insbesondere in der Fettsäureesterkomponente und/oder der Proteinkomponente enthalten; andere Bestandteile des wirkstoffhaltigen Schaums (abgesehen von Wasser) spielen im Regelfall nur eine untergeordnete Rolle für die Stabilisierung des Schaums, so dass häufig der schaumstabilisierende Bestandteil ausschließlich in der Fettsäureesterkomponente und/oder der Proteinkomponente enthalten ist (wenn man von eventuell durch den Wirkstoff auftretenden Effekten absieht - wobei die primäre Aufgabe des Wirkstoffes aber nicht die Schaumstabilisierung sondern die spezifische Verwendung des Schaums ist).

In den meisten Fällen wird der schaumstabilisierende Bestandteil entweder nur durch die Proteinkomponente (bzw. eines Teils derselben) oder durch Fettsäureesterkomponente und Proteinkomponente zusammen zur Verfügung gestellt. Allerdings sind auch fettstabilisierte Schäume prinzipiell denkbar, insbesondere wenn ein Teil des Fettes in kristalliner Form vorliegt. Im Regelfall sind die erfindungsgemäßen Schäume aber stark proteinstabilisiert, wobei der schaumstabilisierende Proteinbestandteil bzw. das schaumstabilisierende Protein dann Aminosäuren bzw. Aminosäuresequenzen aufweist, durch die insbesondere bei der Bildung des Schaums die Grenzfläche zur Luft bzw. dem Gas stabilisiert wird. Die Proteinstruktur des schaumstabilisierenden Proteins weist daher eine entsprechend ausgewogene Anzahl und Anordnung von hydrophoben und hydrophilen Aminosäuren auf, wie sie beispielsweise in Peptiden wie β-Lactoglobulin, α-Lactalbumin und β-Casein vorhanden ist. Als schaumstabilisierende Proteine sind ferner zu nennen: Ei-Albumin, Casein-Hydrolysate, Keratin-Hydrolysate, Soja-Peptones und Soja-Albumin, Molke und Milchserumproteine, Weizenalbumin, Weizenglobulin, Conalbumin und Avidin.

Als schaumstabilisierender Bestandteil der Fettsäureesterkomponente ist beispielsweise Lecithin zu nennen.

Zur Verbesserung der schaumstabilisierenden Eigenschaften der Proteinkomponente kann das proteinhaltige Ausgangsmaterial auch so behandelt werden, dass die schaumstabilisierenden Segmente oder Proteine gezielt angereichert werden. Beispielsweise kann eine enzymatische Hydrolyse des proteinhaltigen Ausgangsmaterials erfolgen, wobei bei niedrigen Hydrolysegraden häufig die Grenzflächeneigenschaften des Proteins ein Maximum annehmen. Ferner kann eine gezielte Fraktionierung des Rohstoffs durchgeführt werden, beispielsweise mittels Filtrationsverfahren oder spezielle Fällungsstrategien. Verbesserte Grenzflächeneigenschaften werden dabei insbesondere in den niedermolekularen Fraktionen erhalten. Beispielsweise können mittels Ultrafiltration Proteine mit einem Molekulargewicht von 1 bis 200 kDa erhalten werden.

Durch die genannten Verfahren ist es somit auch möglich, bei einem gegebenen Proteinausgangsmaterial nicht nur die Schaumstabilisierung zu erhöhen, sondern auch die Molekülgröße so zu erniedrigen, dass beim Auftragen des proteinhaltigen Schaums auf der Haut ein möglichst geringer Abrieb entsteht.

Gemäß einer Ausführungsform ist der Wirkstoff derart in die Fettsäureester eingebracht, dass er in gelöster oder in verkapselter Form vorliegt. Dies hat den Vorteil, dass eine besonders homogene Verteilung des Wirkstoffs in der Fettsäureesterkomponente und damit auch im wirkstoffhaltigen Schaum gewährleistet werden kann. Eine verkapselte Form des Wirkstoffs bietet sich insbesondere dann an, wenn der Wirkstoff an sich nicht besonders lipophil ist und mit einer Hülle die Lipophilie erhöht werden kann. Ferner ist eine Verkapselung z. B. dann sinnvoll, wenn der Wirkstoff durch die Verkapselung eine verbesserte Stabilität besitzt, etwa weil er flüchtig ist oder in unverkapselter Form in unerwünschter Weise reagiert (etwa durch Reaktion mit Luftsauerstoff). Wird der verkapselte wirkstoffhaltige Schaum auf die Haut aufgetragen, so kann er durch das Reiben freigesetzt werden.

Der Wirkstoff kann bereits in der biogenen Fettsäureesterkomponente und/oder der biogenen Proteinkomponente enthalten sein; im Regelfall wird der Wirkstoff aber zugesetzt. Ferner kann der Wirkstoff gasförmig sein, im Regelfall handelt es sich aber um einen Feststoff oder eine Flüssigkeit. Als gasförmiger Wirkstoff kommen beispielsweise lipophile Edelgase wie Xenon oder Radon in Betracht; die erfindungsgemäßen Schäume sind dann beispielsweise im Rahmen der Radon-Therapie oder in der Anästhesie einsetzbar. Xenon und Radon sind in Ölen wie z.B. Olivenöl deutlich besser löslich als in Wasser oder wässrigen Medien. Im Regelfall wird das Edelgas aber nicht ausschließlich in der Fettsäureesterkomponente angereichert sein, sondern auch zumindest einen Teil des Gases darstellen, das nach Verfahrensschritt C) in den Schaumblasen vorliegt.

Gemäß einer Ausführungsform ist der kosmetische oder pharmazeutische Wirkstoff ausgewählt aus einem oder mehreren der Stoffe der Gruppe bestehend aus Arzneistoffen, antibakteriellen Stoffen, antimikrobiellen Stoffen, Antitranspirantien, Antioxidantien, Pflegestoffen, Geruchsabsorbern, Vitaminen, UV-Adsorbern und ätherischen Ölen.

Als (kosmetische) Pflegestoffe sind insbesondere folgende Stoffe zu nennen: Antischuppenwirkstoffe (die der Schuppenbildung entgegen wirken), bräunende Wirkstoffe (die ein Tönen der Haut bewirken ohne die Einwirkung von UV-Strahlen), enthaarende Wirkstoffe, erfrischende Wirkstoffe (die der Haut eine angenehme Frische verleihen), geschmeidig machende Wirkstoffe (die die Haut geschmeidig machen und glätten), feuchthaltende Wirkstoffe (die die Feuchtigkeit der Haut bewahren), Haarfärbemittel, kosmetische Farbstoffe (die kosmetische Mittel und/oder Farbe verleiht), rückfettende Wirkstoffe (die Lipide für das Haar oder die oberen Hautschichten spenden).

Als Wirkstoffe sind insbesondere auch Naturstoffe mit pharmazeutischer oder kosmetischer Wirksamkeit zu nennen. Bei der Verwendung von derartigen Stoffen werden dann also wirkstoffhaltige Schäume erhalten, bei denen nicht nur die Fettsäureesterkomponente und gegebenenfalls auch die Proteinkomponente (im Wesentlichen) biogenen Ursprungs sind sondern auch noch die Wirkstoffe. In dieser Hinsicht sind auch homöopathische Wirkstoffe zu nennen, für die die erfindungsgemäßen Schäume ebenfalls besonders interessant sind, da in der Homöopathie sehr geringe Konzentrationen des Wirkstoffs eine wesentliche Rolle spielen und mit den erfindungsgemäßen wirkstoffhaltigen Schäumen eine besonders geringe Konzentration besonders homogen aufgebracht werden kann, so dass die Gefahr lokaler Überdosierungen effektiv vermieden werden kann. Entsprechendes gilt natürlich auch für Arzneistoffe, die in geringen Konzentrationen appliziert werden sollen. Nur beispielhaft seien nachfolgend einige medizinisch wirksame Naturstoffe genannt, die in den erfindungsgemäßen Schäumen Verwendung finden können: Beta-Sitosterin-Extrakt aus dem afrikanischen Pflaumenbaum (Pygeum africanum) wird für eine begleitende Therapie bei Prostatakrebs eingesetzt, Capsaicin aus der Chilischote wird für die Unterstützung des Bewegungsapparates eingesetzt, Flavonoide und Terpene aus der Ringelblume werden zur Unterstützung von Wundheilungsprozessen eingesetzt.

Generell können als lipohile, insbesondere topische Wirkstoffe, unter anderem Wirkstoffe aus folgenden Klassen eingesetzt werden: Antimykotika, (Lokal-)Analgetika, Hormone (z.B. Cortisol, Estradiol), Sedativa, Virostatika, Antibakterizide, Relaxanze, durchblutungsfördernde Mittel. Ferner sind hinsichtlich der (bio)chemischen Herkunft unter anderem folgende Klassen zu nennen: Alkaloide, Terpene, Flavonoide, Fette und Öle, hydrophobe Proteine und Zuckerderivate.

Als Pflegestoffe sind beispielsweise Panthenol, Vitamin B12 oder Jojoba-Öl (das dann nicht als Fettsäureesterkomponente sondern als Wirkstoff neben einer anderen Fettsäureesterkomponente vorliegt) zu nennen oder auch Vitamin E (das nicht nur zu den Oxidantien zählt, sondern auch als Hautpflegemittel dient, da es freie Radikale abfängt). Als klassische Wirkstoffe sind insbesondere Wirkstoffe zu nennen, die für die topische Anwendung eingesetzt werden. Zu nennen sind etwa Wirkstoffe gegen Schmerzen und Schwellungen oder in der Dermatologie angewendete Wirkstoffe.

Gemäß einer Ausführungsform beträgt der Anteil des Wirkstoffs in dem in Schritt B) erhaltenen Gemisch ≤ 5 Gew.-%, insbesondere ≤ 1 Gew.-%. Als obere Grenze ist häufig die Löslichkeit des Wirkstoffs in der Fettsäureesterkomponente ein Kriterium, wobei - wie ausgeführt - auch eine Verkapselung des Wirkstoffs vorliegen kann. Grundsätzlich ist aber festzustellen, dass die erfindungsgemäßen Schäume nicht nur gewährleisten, dass besonders geringe Konzentrationen des Wirkstoffs aufgebracht werden, sondern auch, dass überhaupt bestimmte Wirkstoffe für Schaumapplikationen zugänglich sind. Durch den relativ hohen Fettsäureesteranteil kann nämlich auch ein besonders hoher Anteil schlechter löslicherer Wirkstoffe in den Schaum eingebracht werden.

In Schritt C) wird anmeldungsgemäß ein fettlösliches Gas ausgewählt aus einem oder mehreren Gasen der Gruppe bestehend aus Stickstoff, Lachgas und Xenon eingebracht. Generell sind Gase geeignet, die den Druck nachliefern. Das eingebrachte Gas kann entweder aus einem oder mehreren der vorstehend genannten Gase bestehen oder diese enthalten. In letzterem Fall kann beispielsweise angereicherte Luft als Gas eingesetzt werden, wobei der Gehalt der fettlöslichen Gase im angereicherten Gas den Gehalt der fettlöslichen Gase in Luft zumindest um 10 Gew.-% (absolut) häufig um 15 Gew.-% und oft um 20 Gew.-% (absolut) übersteigt. Mit Hilfe derartiger fettlöslicher Gase kann die Schaumbildung verbessert werden.

Neben Fettsäureesterkomponente, Proteinkomponente, Wirkstoff und Gas ist in dem erfindungsgemäßen wirkstoffhaltigen Schaum bzw. der Zusammensetzung zu dessen Herstellung (in dem noch kein Gas enthalten ist) vor allem Wasser vorhanden. Der Wasseranteil der Zusammensetzung, die nach Schritt B) erhalten wird, beträgt 30 bis 95 Gew.-% (dementsprechend beträgt der aufsummierte Anteil von Proteinkomponente und Fettsäureesterkomponente 5 bis 70 Gew.-%, wenn man den Wirkstoffgehalt in erster Näherung vernachlässigt). Häufig wird allerdings Wasser in einem Gesamtanteil von 65 bis 95 Gew.-% vorliegen, insbesondere im Bereich 60 bis 70 Gew.-%. (Der aufsummierte Fettsäureesterkomponente- / Proteinkomponente-Anteil beträgt dann - bei Vernachlässigung des Wirkstoffanteils - 5 bis 35 %, insbesondere 20 bis 30 %; allerdings werden häufig der Wirkstoffgehalt und der Gehalt weiterer Zusatzstoffe nicht vernachlässigbar sein, so dass der aufsummierte Anteil der beiden Komponenten im Regelfall niedriger ist und beispielsweise 4 bis 30 Gew.-%, insbesondere 15 bis 25 Gew.-% beträgt).

Statt Wasser kann grundsätzlich auch eine Mischung von verschiedenen Flüssigkeiten vorliegen; im Regelfall liegt aber - abgesehen von gegebenenfalls flüssig vorliegender Proteinkomponente, Fettsäureesterkomponente und Wirkstoffkomponente keine weitere Flüssigkeit als Wasser vor.

Gemäß einer weiteren Ausführungsform werden in Schritt B) neben Proteinkomponente und Fettsäureesterkomponente mit Wirkstoff sowie Wasser auch noch Hilfsstoffe zugemischt. Als typische Hilfsstoffe sind insbesondere Verdicker, Polysaccharide, Konservierungsmittel, Peeling-Zusätze und/oder Trennmittel zu nennen.

Für den anmeldungsgemäß stets enthaltenen Verdicker kommen beispielsweise Hydroxymethylpropylcellulose, Carboxymethylstärke, Methylcellulose, Guaran, Carubin, Carrageene, also insbesondere Polysaccharidderivate wie Poysaccharidether und -ester in Betracht. Ferner sind Johannisbrotkernmehl und andere in der Lebensmittelindustrie verwendete Verdicker zu nennen. Mittels der Verdicker kann eine Erhöhung der Stabilität der Schäume realisiert werden. Gemäß einer Ausführungsform kann der schaumstabilisierende Bestandteil somit auch einerseits aus einem in der Proteinkomponente enthaltenen schaumstabilisierenden Protein und/oder einem in der Fettsäureesterkomponente enthaltenen schaumstabilisierenden Bestandteil und andererseits aus dem Verdicker, insbesondere einem der zuvor explizit aufgeführten Verdicker, bestehen.

Als Konservierungsmittel ist beispielsweise Vitamin E-Acetat zu nennen, das einen Oxidationsschutz bietet (allerdings auch als Pflegestoff für die Haut und damit als Wirkstoff zu nennen ist).

Ferner sind als Hilfsstoffe maskierende Stoffe, die den Grundgeruch der Masse aus Fettsäureesterkomponente, Proteinkomponente und Wirkstoff verringern oder hemmen können.

Nicht als Hilfsstoffe bzw. generell nicht enthalten sind - wie bereits ausgeführt - synthetische Tenside. Auch auf den Zusatz teilsynthetischer Tenside wird verzichtet. Natürliche Tenside können zwar enthalten sein, im Regelfall werden diese aber nicht absichtlich zugesetzt, sondern sind im Regelfall nur in der Fettsäureesterkomponente und/oder der Proteinkomponente enthalten. Ferner ist auszuführen, dass die erfindungsgemäßen Schäume im Regelfall auch keine Nanopartikel enthalten, wie sie beispielsweise Pickering-Emulsionen enthalten.

Bezüglich des Kohlenhydratanteils, der zwar grundsätzlich - wie ausgeführt - vorliegen kann, ist zu beachten, dass der Anteil von Kohlenhydraten, die in der Lebensmittelindustrie zum Süßen eingesetzt werden üblicherweise vernachlässigbar ist (also bezogen auf das Gemisch das nach Schritt B) erhalten wird, kleiner 1 % ist.

Generell ist der Anteil von Kohlenhydraten normalerweise geringer als der Gewichtsanteil der Proteinkomponente, insbesondere auch geringer als der des Proteinanteils der Protein komponente.

Der Anteil von zusätzlichen Hilfsstoffen an dem Gemisch, dass nach Schritt B) erhalten wird kann insbesondere 0 bis 20 Gew.-% betragen, liegt üblicherweise aber im Bereich 2 bis 5 Gew.-%.

Im Folgenden wird das erfindungsgemäße Verfahren - ohne Einschränkung der Allgemeinheit - noch näher beschrieben:
Für den wirkstoffhaltigen Schaum wird zunächst die Proteinkomponente, die insbesondere so ausgewählt wird, dass sie den wirkstoffhaltigen Schaum später stabilisieren kann, in Wasser homogenisiert. Als Proteinkomponente können beispielsweise Weizenprotein-, Reisprotein-, Sojaprotein-, Kartoffelprotein- und Milchprotein-haltige Proteinkomponenten verwendet werden. Diese Proteine können dann gegebenenfalls zur Verbesserung ihrer Eigenschaften chemisch modifiziert oder auch hydrolysiert oder zerkleinert werden. Grundsätzlich kann auch ein bereits wässrig vorliegendes Protein bzw. eine wässrig vorliegende Proteinkomponente Verwendung finden, wobei dann für die vorstehenden Angaben der Gewichtsprozente zunächst der Wassergehalt zu bestimmen und abzuziehen ist.

Im nächsten Schritt werden zu dem Homogenisat aus Proteinkomponente und Wasser die ggf. zuzugebenden Hilfsstoffe, beispielsweise ein Verdicker und ein Wirkstoff und Konservierungsmittel wie Vitamin-E-Acetat zugegeben und ebenfalls homogen verteilt. Schließlich wird noch die Fettsäureesterkomponente, insbesondere ein Öl, zu der gut homogenisierten Mischung hinzugegeben und verteilt. Der Wirkstoff kann bereits in dieser Fettsäureesterkomponente vorliegen, kann aber auch in das Gemisch eingebracht werden bzw. bereits zu einem früheren Zeitpunkt eingebracht sein. Nachdem es sich häufig allerdings um lipophile Stoffe handelt, wird üblicherweise der Wirkstoff zusammen mit der Fettsäureesterkomponente dem Gemisch zugegeben.

Um aus dem vorstehend beschriebenen Gemisch einen Schaum zu generieren wird die Emulsion beispielsweise mit einem möglichst fettlöslichen Treibgas in einem Druckgasgefäß unter Druck gesetzt. Das Gas kann dabei insbesondere unter einem Druck von 1 bis 15 hPa, bevorzugt 5 bis 9 hPa eingebracht werden. Der fertige Schaum kann üblicherweise nach kräftigem Schütteln aus dem Druckgasgefäß entnommen werden bzw. aus diesem aufgebracht werden.

Insbesondere auch das Einbringen des Gases ist nicht auf die bevorstehend beschriebene Art beschränkt, vielmehr sind auch andere Verfahren denkbar, die dem Fachmann bekannt sind.

Die Erfindung betrifft auch eine Zusammensetzung, die zur Herstellung des wirkstoffhaltigen Schaums dienen kann, und die insbesondere nur noch das Gas eingebracht werden muss. Diese Zusammensetzung umfasst eine Fettsäureesterkomponente, eine Proteinkomponente, einen Wirkstoff und Wasser, wobei die Fettsäureesterkomponente und/oder die Proteinkomponente einen schaumstoffstabilisierenden Bestandteil enthalten, der ein in der Proteinkomponente enthaltenes schaumstabilisierendes Protein und/oder einen in der Fettsäureester enthaltenen schaumstabilisierenden Bestandteil umfasst oder daraus besteht. In der Fettsäureesterkomponente liegt der Wirkstoff, üblicherweise in gelöster oder verkapselter Form vor, wobei es sich bei dem Wirkstoff um einen pharmazeutischen oder kosmetischen Wirkstoff handelt. Zumindest die Fettsäureesterkomponente ist dabei im Wesentlichen biogenen Ursprungs. Ferner liegen die Proteinkomponente und die Fettsäureesterkomponente in einem Gewichtsverhältnis von 1 : 10 bis 10 : 1 vor. Schließlich beträgt das Gewichtsverhältnis zwischen Wasser und den aufsummierten Anteilen der Proteinkomponente und der Fettsäureesterkomponente 1 : 1 bis 20 : 1 (Wasser liegt also im gewichtsmäßigen Überschuss vor. Der Anteil der Fettsäureesterkomponente in der Zusammensetzung beträgt üblicherweise mehr als 3 Gew.-%.

Mit der erfindungsgemäßen Zusammensetzung ist es in einfacher Weise möglich, wirkstoffhaltige Schäume zu erzeugen, die für verschiedenste Anwendungen, insbesondere für das Auftragen auf Körpergewebe geeignet sind.

Die vorliegende Erfindung betrifft schließlich auch einen wirkstoffhaltigen Schaum, der gemäß den näher beschriebenen Verfahren herstellbar ist, bzw. durch Einbringen eines Gases in die vorstehend beschriebene Zusammensetzung erhältlich ist. Der wirkstoffhaltige Schaum hat üblicherweise ein glänzendes Erscheinungsbild und weist eine relativ hohe Schaumstoffstabilität auf, so dass vielfach ein Zerfliesen des Schaumes frühestens nach zwanzig Minuten zu beobachten ist.

Gemäß einer Ausführungsform ist der Volumenanteil der nicht gasförmigen Bestandteile im frisch hergestellten Schaum maximal 35%, insbesondere 10 bis 35 %, beispielsweise 20 bis 30%. Es wird also ein Schaum erhalten, der gegenüber der noch nicht aufgeschäumten Formulierung zumindest ein etwa 3 mal größeres Volumen hat, so dass dementsprechend auch eine deutlich verbesserte Applizierbarkeit des Schaums gegenüber der gasfreien Formulierung gegeben ist.

Die erfindungsgemäßen wirkstoffhaltigen Schäume können grundsätzlich für alle kosmetischen oder medizinischen Anwendungen eingesetzt werden, in denen Schäume sinnvoll sind. Insbesondere ist aber das Aufbringen auf Körpergewebe zu nennen, wobei die Körpergewebe nicht auf Haut beschränkt sind sondern auch Schleimhäute und durch Verletzungen oder medizinische, insbesondere chirurgische, Eingriffe offen liegendes Körpergewebe zu nennen. In diesem Zusammenhang ist nochmals festzustellen, dass sich die erfindungsgemäßen Schäume besonders durch ihre gewebeverträgliche Zusammensetzung auszeichnen, da sie weder synthetische Tenside noch Nanopartikel oder ähnliches enthalten und dementsprechend im Wesentlichen aus biogenen Materialien zusammengesetzt sind, so dass eine besonders schonende Applikation möglich ist. Zu dem wird durch die Schaumstruktur gewährleistet, dass eine besonders gleichmäßige Applikation in besonders geringen Konzentrationen möglich ist und lokale Überdosierungen wirksam vermieden werden können.

Weitere Vorteile und vorteilhafte Ausführungsformen und Weiterbildungen der Erfindung ergeben sich im Folgenden - ohne Einschränkung der Allgemeinheit - aus den Beispielen.

### Beispiel 1

Der wirkstoffhaltige Schaum wird aus folgenden Bestandteilen zusammengesetzt:

| | |
|---|---|
| 30 g | Total Milk Protein (Firma Milei GmbH mit einem Proteingehalt von 80 % in der Trockenmasse, einem Lactosegehalt von 9%, einem Mineraliengehalt von 7%, einem Fettgehalt von 1%, einem Wassergehalt von 6% (jeweils Gew.-%) und einem pH-Wert von 6,9) |
| 200 g | Wasser, bidestilliert |
| 5 g | Hydroxymethylpropylcellulose (4000 Tegocelfluid der Firma Evonik) |
| 5 g | Vitamin-E-Acetat |
| 50 g | Sonnenblumenöl der Marke Thomy |
| 20 Tropfen | Mandarinenöl (als maskierende Komponente) |
| 8 g | Lachgas |

Das Protein wird eingewogen, Wasser hinzugegeben und die Mischung homogenisiert. Anschließend wird der Verdicker zugesetzt und es erfolgt eine erneute Homogenisierung. Schließlich werden die verbleibenden Komponenten, Vitamin E-Acetat (als lipophiler Wirkstoff), Sonnenblumenöl und Mandarinenöl zugemischt.

Die erhaltene Emulsion wird in ein Druckgefäß gegeben und mit 8 g Lachgas unter Druck gesetzt. Nach kräftigem Schütteln und einer kurzen Ruhezeit kann der fertige Schaum dem Druckbehälter entnommen werden. Der Volumenanteil der nicht gasförmigen Bestandteile beträgt etwa 25 %.

Der erhaltene Schaum ist weiß und besitzt einen Geruch nach Mandarinenöl.

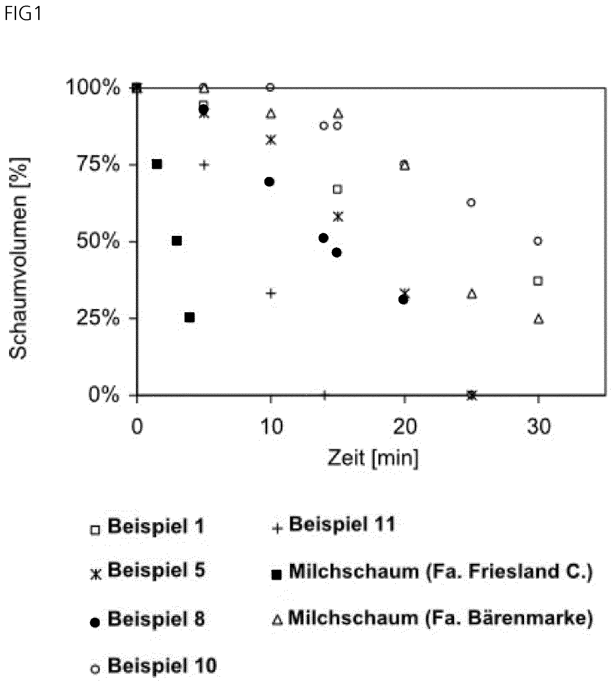

In einem Drainageversuch wurde der erfindungsgemäße Schaum mit einem kommerziell erhältlichem Chantibic - Milchschaum mit 0 % Fett (Firma Friesland Campina. Lumen / Belgien) verglichen, der nur Verdicker (Xanthan) aber keinen Emulgator enthält. Figur 1 zeigt die zeitliche Abnahme des Schaumvolumens, wobei der Schaum nach Beispiel 1 (ungefüllte Quadrate) eine deutlich höhere Stabilität als das käufliche Produkt (gefüllte Quadrate) aufweist. Die erfindungsgemäße Öl / Protein-Mischung weist also gegenüber dem kommerziellen Produkt mit einem wesentlich niedrigeren Fett-Gehalt deutliche Vorteile auf. Vergleichbare Stabilitäten werden nur mit einem kommerziell erhältlichem Milchschaum (Fa. Bärenmarke) erzielt, der allerdings zusätzlich Emulgatoren (E471, E472) und verschiedene Verdicker enthält (Dreiecke).

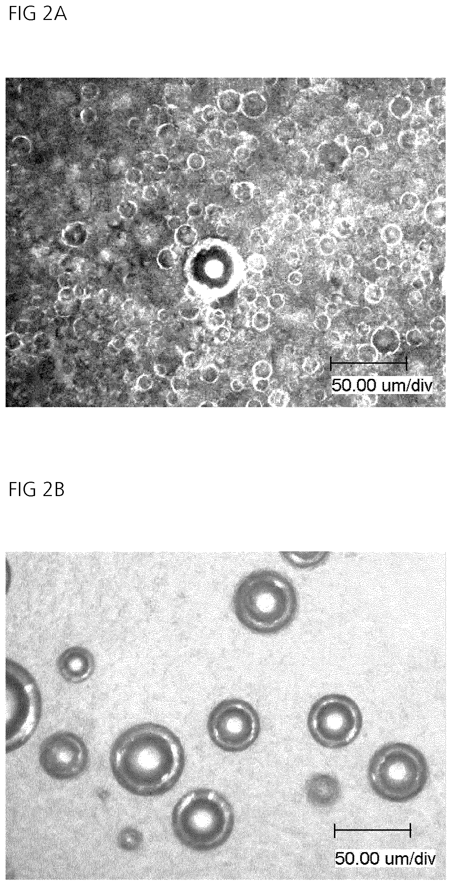

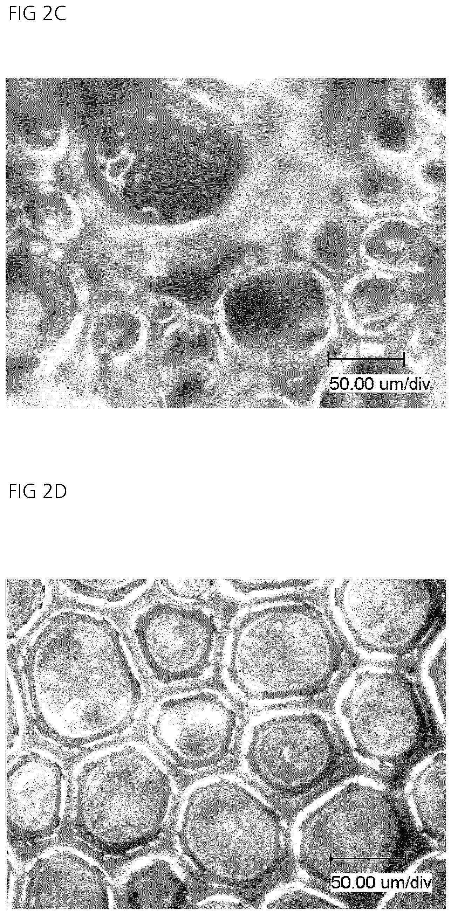

Die Figuren 2A bis 2D zeigen lichtmikroskopische Aufnahme verschiedener Schäume. Fig 2A zeigt den erfindungsgemäßen Schaum, Fig 2B den fettfreien Milchschaum der Firma Friesland Campina aus Figur 1, Fig 2C einen mittels eines modifizierten Proteins stabilisierten Schaum (Fußpflegeschaum der Firma Allpresan) und Fig 2D einen Trockenschaum (Rasierschaum der Marke Insana, Firma Rossmann - enthält synthetische Tenside). Auf Grund des verwendeten Objektträgers wurden insbesondere in Fig 2A und Fig 2B Gasblasen in gewissem Umfang zerstört, es lässt sich allerdings sehr schön in Fig. 2A erkennen, dass eine besonders homogene Verteilung der Fettkomponente im Schaum vorliegt. Die Größe der Fett"tröpfchen" ist heterogen und reicht von 0,5 bis 30 µm (Durchmesser). Der mittlere Durchmesser der Fett"tröpfchen" beträgt etwa 5 bis 15 µm (ermittelt durch Auswertung der lichtmikroskopischen Aufnahme).

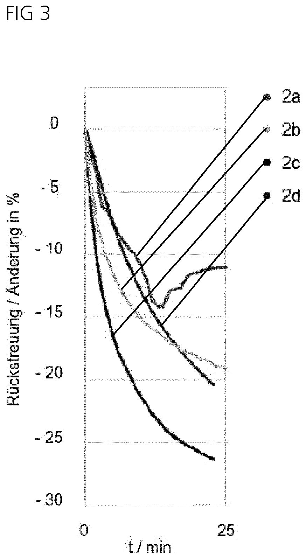

Lichtstreuexperimente erlauben eine Aussage über die Schaumstabilität. Eine Verringerung der Rückstreuintensität ist auf eine Koagulation der Gasblasen zurückzuführen. Figur 3 zeigt die Abnahme der Rückstreuintensität in Abhängigkeit der Zeit bezogen auf die Ausgangsintensität für die Schäume gemäß Fig. 2A bis 2D, wobei 2a den Schaum gemäß Fig 2A bezeichnet, 2b den Schaum gemäß Fig 2B, 2c den Schaum gemäß Fig 2C und 2d den Schaum gemäß Fig 2D.

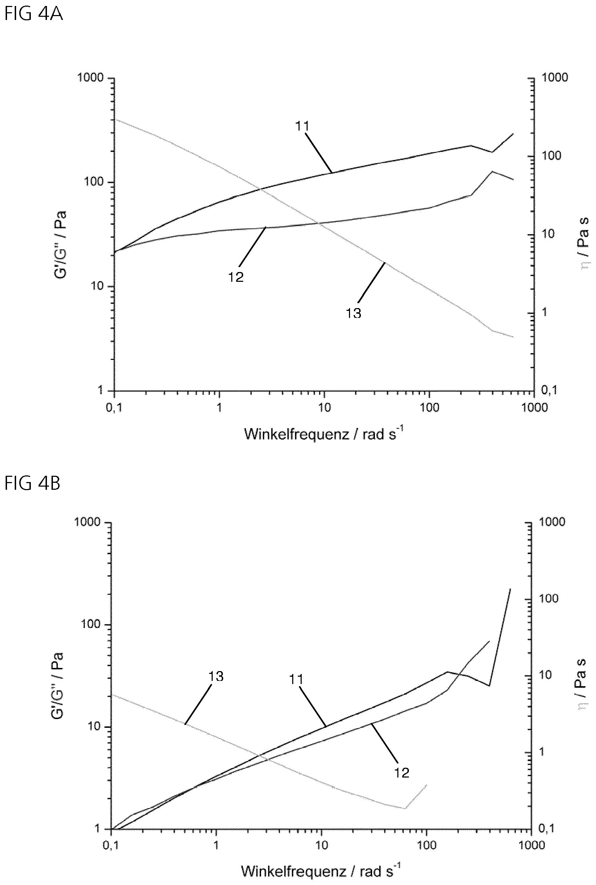

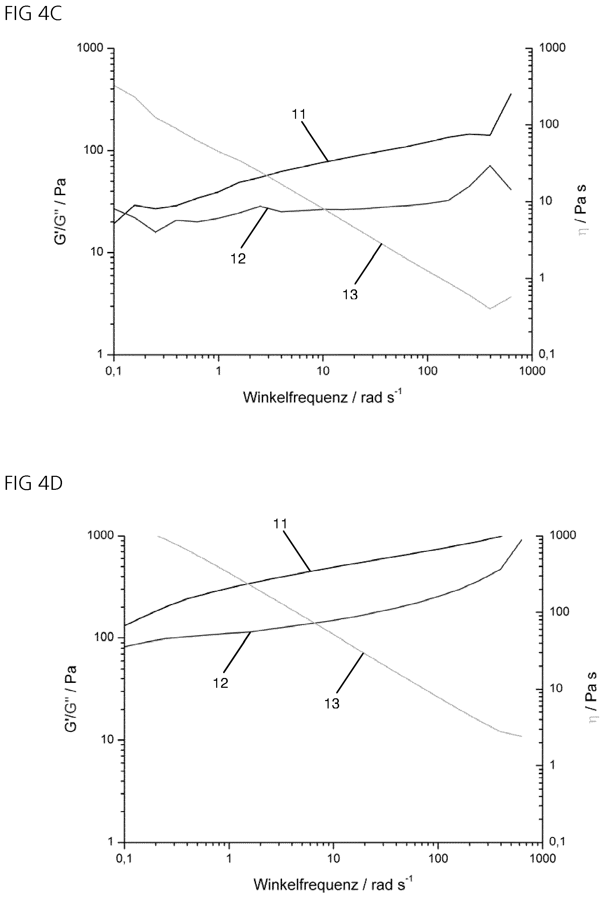

Rheologische Experimente beschreiben das Fließverhalten von Schäumen. Über frequenzabhängige Messungen des Speichermoduls G' und des Verlustmoduls G" im viskoelastischen Bereich des jeweiligen Schaumes lassen sich die Fließeigenschaften unterschiedlicher Schäume miteinander vergleichen. Aus beiden Modulen kann die (dynamische) Viskosität η berechnet werden. Figur 4A bis 4D zeigen Speichermodul G' (11), Verlustmodul G" (12) und (dynamische) Viskosität η (13) in Abhängigkeit der Winkelfrequenz für die Schäume gemäß Fig. 2A bis 2D, wobei 4A den Schaum gemäß Fig 2A bezeichnet, 4B den Schaum gemäß Fig 2B, 4C den Schaum gemäß Fig 2C und 4D den Schaum gemäß Fig 2D.

### Beispiel 2

Der Schaum enthält folgende Bestandteile

| | |
|---|---|
| 50 g | Sprühmagermilchpulver, aufkonzentriert (Firma Nöll & Co. GmbH, Büren mit einem Milchzuckergehalt von ca. 52 %, Proteingehalt von mindestens 34%, Milchmineraliengehalt von ca. 7,5 - 8,0 %, Milchfettgehalt von max. 1,0 %, Wassergehalt von max. 5,0 % (jeweils Gew.-%) und einem pH-Wert von ca. 6,6 |
| 200g | Wasser, bidestilliert |
| 3 g | Hydroymethylpropylcellulose (4000 Tegocelfluid der Firma Evonik) |
| 30 g | Sonnenblumenöl der Marke Thomy |
| 8 g | Lachgas |

Die Zusammensetzung wird wie im Beispiel 1 verarbeitet; man erhält ebenfalls einen Schaum mit deutlich erhöhter Stabilität. Im Vergleich zu Beispiel 1 ist hier der Ölanteil etwas erniedrigt und der Proteinanteil etwas erhöht; die erzielten Ergebnisse sind aber vergleichbar. Der Schaum aus Beispiel 2 enthält zwar hier keinen Wirkstoff, allerdings ist es für den Fachmann selbstverständlich, dass Wirkstoffe, insbesondere in geringen Konzentrationen zu keinen deutlich anderen Ergebnissen führen.

### Beispiel 3

Der Schaum enthält folgende Bestandteile

| | |
|---|---|
| 30 g | Weizenprotein, desaminiert (Firma Cargill Company) |
| 200 g | Wasser, bidestilliert |
| 3 g | Hydroxymethylpropylcellulose (4000 Tegocelfluid der Firma Evonik) |
| 5 g | Vitamin-E-Acetat |
| 50 g | Sonnenblumenöl der Marke Thomy |
| 20 Tropfen | Mandarinenöl (als maskierende Komponente) |
| 8 g | Lachgas |

Die Zusammensetzung wird wie im Beispiel 1 verarbeitet; man erhält einen Schaum mit gegenüber dem Schaum aus Beispiel 1 deutlich erhöhter Stabilität aber vergleichbarer Schaumstruktur. Der erhaltene Schaum ist leicht braun und besitzt einen neutralen Geruch.

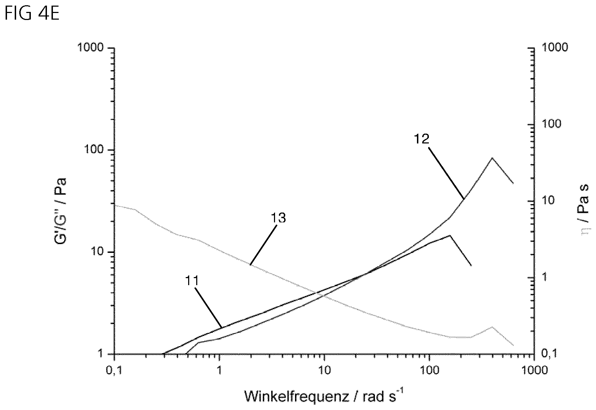

Figur 4E zeigt Speichermodul G' (11), Verlustmodul G" (12) und (dynamische) Viskosität η (13) in Abhängigkeit der Winkelfrequenz für den erhaltenen Schaum.

### Beispiel 4

Der Schaum enthält folgende Bestandteile

| | |
|---|---|
| 30 g | Reisprotein (Remypro N80+ der Firma Remy Industries N.V., Proteingehalt mindestens 79 Gew.-% der Trockenmasse, Fettgehalt max. 5 Gew-% der Trockenmasse) |
| 200 g | Wasser, bidestilliert |
| 5 g | Hydroxymethylpropylcellulose (4000 Tegocelfluid der Firma Evonik) |
| 5 g | Vitamin-E-Acetat |
| 50 g | Sonnenblumenöl der Marke Thomy |
| 20 Tropfen | Mandarinenöl (als maskierende Komponente) |
| 8 g | Lachgas |

Die Zusammensetzung wird wie im Beispiel 1 verarbeitet; man erhält einen Schaum mit gegenüber dem Schaum aus Beispiel 1 verringerter Stabilität aber vergleichbarer Schaumstruktur. Der erhaltene Schaum ist beige und besitzt einen gewissen Reis-Geruch. Ferner zeigt der Schaum einen ausgeprägten Peeling-Effekt.

### Beispiel 5

Der Schaum enthält folgende Bestandteile

| | |
|---|---|
| 30 g | Total Milk Protein wie in Beispiel 1 definiert |
| 200 g | Wasser, bidestilliert |
| 0,9 g | Hydroxymethylpropylcellulose |
| 50 g | Sonnenblumenöl der Marke Horeca |
| 16 g | Lachgas |

Beispiel 5 unterscheidet sich von Beispiel 1 dahingehend, dass deutlich weniger Verdicker verwendet wurde und mit der doppelten Menge Gas aufgeschäumt wurde. Darüber hinaus wurde auf den Wirkstoff und die maskierende Komponente verzichtet. Im Vergleich zum Beispiel 1 ist die Hydroxymethylpropylcellulose nicht vorformuliert und wird wie folgt angesetzt: 20 ml des Wassers werden auf 80°C erhitzt und die Hydroxypropylmethylcellulose langsam unter ständigem Rühren hinzugeben bis sie vollständig gelöst ist, weitere 30 ml Wasser hinzugegeben und 30 min nachgerührt.

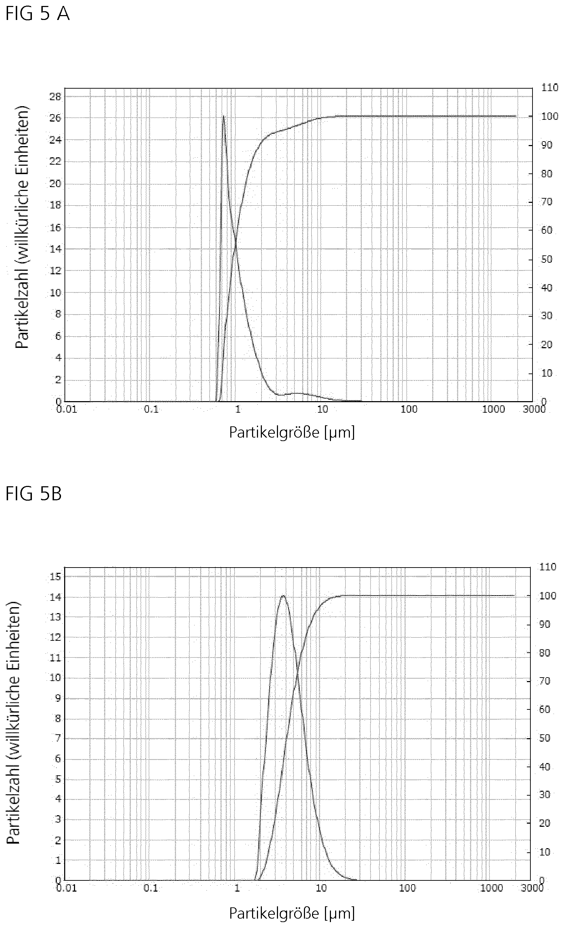

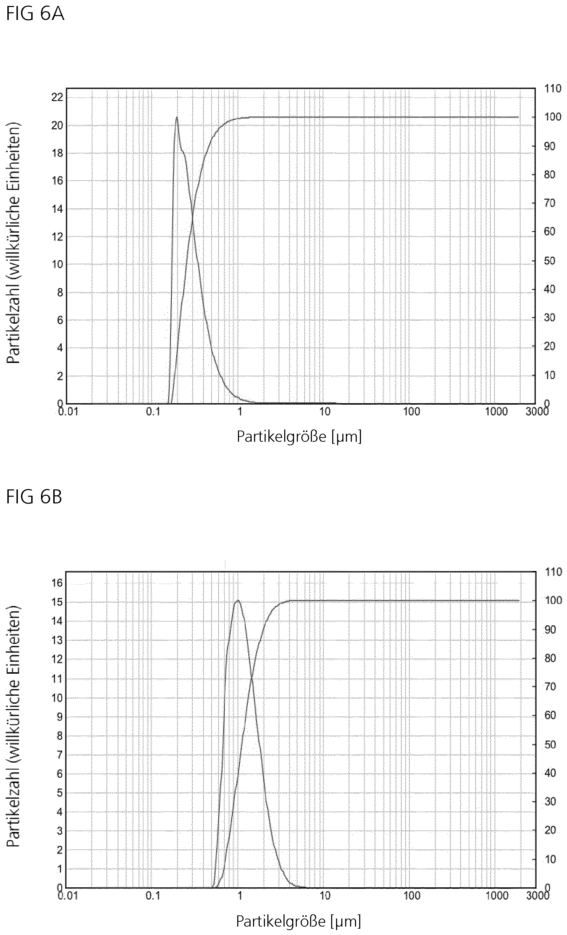

Das Protein wird eingewogen, 150 ml Wasser, das Sonnenblumenöl und die vorformulierte Hydroxymethylpropylcellulose hinzugegeben und die Mischung homogenisiert. Figur 5A zeigt die mittels dynamischer Lichtstreuung ermittelte Partikelgrößenverteilung (d.h. die Tröpfchengrößen der Fettsäureesterkomponente) für die erhaltene Emulsion. Die mittlere Partikelgröße beträgt 9,5 µm und ist damit größer als in Sprühsahne (1,9 µm - Marke Milboa der Firma Lidl), für die die Partikelgrößenverteilung in Figur 6A gezeigt ist. Milchschaum (Firma Bärenmarke) hat ebenfalls eine kleinere mittlere Partikelgröße (2,8 µm -Partikelgrößenverteilung in Figur 6B). Die erhaltene Emulsion wird in ein Druckgefäß gegeben und mit 16 g Lachgas unter Druck gesetzt. Nach kräftigem Schütteln und einer kurzen Ruhezeit kann der fertige Schaum dem Druckbehälter entnommen werden. Der Volumenanteil der nicht gasförmigen Bestandteile beträgt etwa 12 %.

Man erhält einen Schaum mit gegenüber dem Schaum aus Beispiel 1 vergleichbarer Schaumstruktur. Der erhaltene Schaum ist weiß und besitzt einen leichten Milch-Geruch. Der höhere Gasanteil führt zu einem leichteren Schaum. Figur 1 (Sterne) zeigt eine etwas geringere Schaumstabilität als bei Beispiel 1.

### Beispiel 6

Der Schaum enthält folgende Bestandteile

| | |
|---|---|
| 30 g | Total Milk Protein wie in Beispiel 1 definiert |
| 200 g | Wasser, bidestilliert |
| 0,9 g | Hydroxymethylpropylcellulose |
| 100 g | Sonnenblumenöl der Marke Horeca |
| 16 g | Lachgas |

Beispiel 6 unterscheidet sich von Beispiel 5 dahingehend, dass die doppelte Menge Sonnenblumenöl verwendet wurde. Die Zusammensetzung wird wie im Beispiel 5 verarbeitet; man erhält einen Schaum mit gegenüber dem Schaum aus Beispiel 5 vergleichbarer Stabilität, Schaumstruktur und Gasvolumenanteil. Der erhaltene Schaum ist weiß und besitzt einen leichten Milch-Geruch. Der Volumenanteil der nicht gasförmigen Bestandteile beträgt etwa 12 %. Figur 5B zeigt die Partikelgrößenverteilung für die dem Schaum zugrundeliegende Emulsion. Die mittlere Partikelgröße beträgt 7,8 µm und ist kleiner als bei Beispiel 5.

### Beispiel 7

Der Schaum enthält folgende Bestandteile

| | |
|---|---|
| 30 g | Total Milk Protein wie in Beispiel 1 definiert |
| 200 g | Wasser, bidestilliert |
| 0,9 g | Hydroxymethylpropylcellulose |
| 25 g | Natives Olivenöl Extra der Marke Primadonna |
| 16 g | Lachgas |

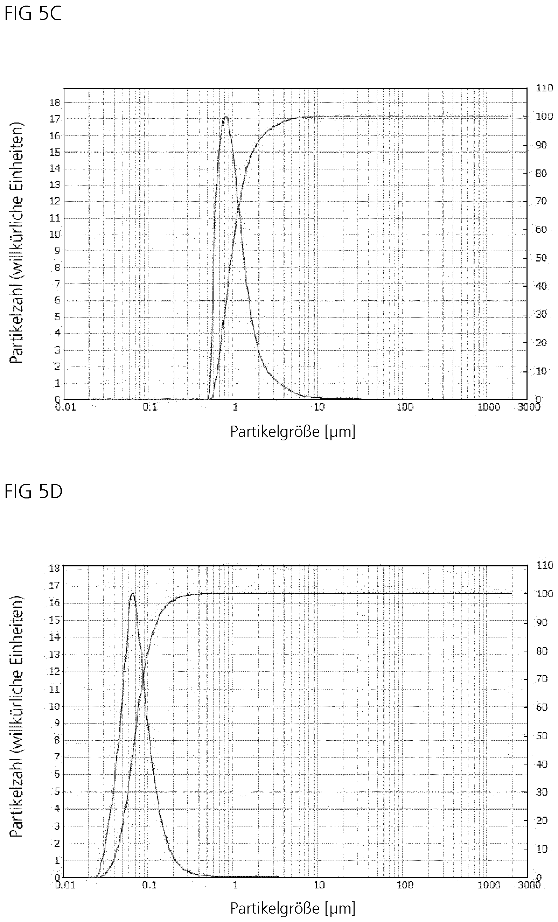

Beispiel 7 unterscheidet sich von Beispiel 5 dahingehend, dass nur die halbe Menge Öl und zwar Olivenöl statt Sonnenblumenöl verwendet wurde. Die Zusammensetzung wird wie im Beispiel 5 verarbeitet; man erhält einen Schaum mit gegenüber dem Schaum aus Beispiel 5 vergleichbarer Stabilität, Schaumstruktur und Gasvolumenanteil. Der erhaltene Schaum ist weiß/leicht grünlich und besitzt keinen markanten Eigengeruch. Figur 5C zeigt die Partikelgrößenverteilung für die dem Schaum zugrundeliegende Emulsion. Die mittlere Partikelgröße beträgt 5,6 µm ist deutlich kleiner als bei Beispiel 5.

### Beispiel 8

Der Schaum enthält folgende Bestandteile

| | |
|---|---|
| 30 g | Total Milk Protein wie in Beispiel 1 definiert |
| 200 g | Wasser, bidestilliert |
| 5 g | Hydroxymethylpropylcellulose (4000 Tegocelfluid der Firma Evonik) |
| 5 g | Vitamin-E-Acetat |
| 50 g | Rapskernöl "Vielseitig" der Marke Teutoburger Ölmühle |
| 20 Tropfen | Mandarinenöl (als maskierende Komponente) |
| 8 g | Lachgas |

Die Zusammensetzung wird wie im Beispiel 1 verarbeitet; man erhält einen Schaum mit gegenüber dem Schaum aus Beispiel 1 vergleichbarer Schaumstruktur und etwas geringerer Stabilität (Figur 1 - gefüllte Kreise). Der erhaltene Schaum ist beige und besitzt einen leichten Milch-Geruch. Figur 5D zeigt die Partikelgrößenverteilung für die dem Schaum zugrundeliegende Emulsion. Die mittlere Partikelgröße beträgt 0,5 µm und ist damit deutlich kleiner als in den vorhergehenden Beispielen 5 bis 7.

### Beispiel 9

Der Schaum enthält folgende Bestandteile

| | |
|---|---|
| 30 g | Weizenprotein, natur (Firma Cargill Company) |
| 200 g | Wasser, bidestilliert |

| | |
|---|---|
| 5 g | Hydroxymethylpropylcellulose (4000 Tegocelfluid der Firma Evonik) |
| 5 g | Vitamin-E-Acetat |
| 50 g | Rapskernöl "Vielseitig" der Marke Teutoburger Ölmühle |
| 20 Tropfen | Mandarinenöl (als maskierende Komponente) |
| 8 g | Lachgas |

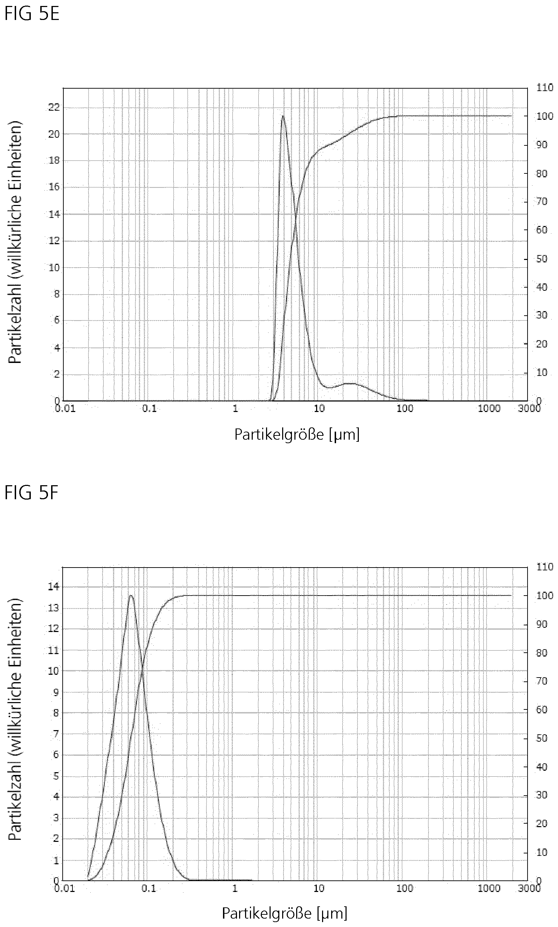

Die Zusammensetzung wird wie im Beispiel 1 verarbeitet; man erhält einen Schaum mit gegenüber dem Schaum aus Beispiel 1 vergleichbarer Schaumstruktur. Der erhaltene Schaum ist beige und besitzt einen deutlichen Weizenprotein-Geruch. Der Volumenanteil der nicht gasförmigen Bestandteile beträgt etwa 22 %. Figur 5E zeigt die Partikelgrößenverteilung für die dem Schaum zugrundeliegende Emulsion. Die mittlere Partikelgröße beträgt 51,6 µm.

### Beispiel 10

Der Schaum enthält folgende Bestandteile

| | |
|---|---|
| 30 g | Weizenprotein, desaminiert (Firma Cargill Company) |
| 200 g | Wasser, bidestilliert |
| 3 g | Hydroxymethylpropylcellulose (4000 Tegocelfluid der Firma Evonik) |
| 5 g | Vitamin-E-Acetat |
| 50 g | Rapskernöl "Vielseitig" der Marke Teutoburger Ölmühle |
| 20 Tropfen | Mandarinenöl (als maskierende Komponente) |
| 8 g | Lachgas |

Beispiel 10 unterscheidet sich von Beispiel 9 dahingehend, dass weniger Verdicker verwendet wurde. Die Zusammensetzung wird wie im Beispiel 1 verarbeitet; man erhält einen Schaum mit gegenüber dem Schaum aus Beispiel 1 vergleichbarer Schaumstruktur und sogar etwas erhöhter Stabilität (Figur 1 - ungefüllte Kreise). Der erhaltene Schaum ist beige und besitzt einen deutlichen Weizenprotein-Geruch. Figur 5F zeigt die Partikelgrößenverteilung für die dem Schaum zugrundeliegende Emulsion. Die mittlere Partikelgröße beträgt 0,2 µm und ist deutlich kleiner als bei Beispiel 9.

### Beispiel 11

Der Schaum enthält folgende Bestandteile

| | |
|---|---|
| 30 g | Total Milk Protein wie in Beispiel 1 definiert |
| 200 g | Wasser, bidestilliert |
| 0,9 g | Hydroxymethylpropylcellulose |
| 50 g | Sonnenblumenöl der Marke Horeca |
| 8 g | Lachgas |

Beispiel 11 unterscheidet sich von Beispiel 5 dahingehend, dass weniger Gas zum Aufschäumen verwendet wurde. Die Zusammensetzung wird wie im Beispiel 1 verarbeitet; man erhält einen Schaum mit gegenüber dem Schaum aus Beispiel 1 vergleichbarer Schaumstruktur und Stabilität (Figur 1). Der erhaltene Schaum ist beige und besitzt einen deutlichen Weizenprotein-Geruch.

Die Zusammensetzung wird wie im Beispiel 5 verarbeitet; man erhält einen Schaum (Figur 1, Kreuze) mit gegenüber dem Schaum aus Beispiel 5 geringerer Stabilität. Der erhaltene Schaum ist weiß und besitzt einen leichten Milch-Geruch. Der Volumenanteil der nicht gasförmigen Bestandteile beträgt etwa 25 %. Figur 5A zeigt die Partikelgrößenverteilung.

Die Erfindung ist nicht durch die Beschreibungen an Hand der Ausführungsbeispiele beschränkt.

## Patentansprüche

1. Verfahren zur Herstellung eines wirkstoffhaltigen Schaums, der ein Protein sowie ein Fett und/oder ein Wachs umfasst, mit folgenden Schritten:
A) Bereitstellung des Fetts und/oder des Wachses (Fettsäureesterkomponente) und der Proteinkomponente, wobei
- die Fettsäureesterkomponente und/oder die Proteinkomponente einen schaumstabilisierenden Bestandteil enthalten, der ein in der Proteinkomponente enthaltenes schaumstabilisierendes Protein und/oder einen in der Fettsäureesterkomponente enthaltenen schaumstabilisierenden Bestandteil umfasst oder daraus besteht,
- die Proteinkomponente und die Fettsäureesterkomponente in einem Gewichtsverhältnis von 1:10 bis 10:1 vorliegen,
- die Fettsäureesterkomponente aus einem Pflanzenöl oder einem pflanzlichen Wachs besteht
- wobei die Proteinkomponente ein pflanzliches Protein, insbesondere ein Getreideprotein, ein Hülsenfruchtprotein oder ein Kartoffelprotein, oder ein Eierprotein oder ein Milchprotein umfasst oder daraus besteht,
- in die Fettsäureesterkomponente mindestens ein pharmazeutischer und/oder kosmetischer Wirkstoff eingebracht ist, eingebracht wird oder in Schritt C) einbringbar ist,
B) Mischen der Proteinkomponente und der Fettsäureesterkomponente unter Zusatz eines Verdickers und unter Zusatz von Wasser, so dass Wasser und die summierten Anteile von Proteinkomponente und Fettsäureesterkomponente in einem Gewichtsverhältnis von 1:1 bis 20:1 vorliegen und wobei der Anteil der Fettsäureesterkomponente im entstandenen Gemisch größer 3 Gew.-% ist,
wobei keine synthetischen oder teilsynthetischen Tenside zugemischt werden,
C) Einbringen eines fettlöslichen Gases, das ausgewählt ist aus Stickstoff, Lachgas, Xenon und Gemischen hiervon in das nach Schritt B) erhaltene Gemisch, so dass ein Schaum entsteht.

2. Verfahren nach einem der vorhergehenden Ansprüche, wobei die Fettsäureesterkomponente ein Öl oder ein Wachs ist, das bei 37 °C flüssig ist.

3. Verfahren nach einem der vorhergehenden Ansprüche, wobei der Anteil der Fettsäureesterkomponente in dem in Schritt B) erhaltenen Gemisch 5 bis 50 Gew.-% beträgt, und insbesondere zwischen 10 und 35 Gew.-% liegt.

4. Verfahren nach einem der vorhergehenden Ansprüche, wobei die zumindest 90 Gew.-% der in der Proteinkomponente enthaltenen Proteine ein Molekulargewicht ≤ 10 kDa aufweisen.

5. Verfahren nach einem der vorhergehenden Ansprüche, wobei als Wirkstoff zumindest ein Arzneistoff, ein antibakterieller Stoff, ein antimikrobieller Stoff, ein Antitranspirant, ein Antoxidans, ein Pflegestoff, ein Geruchsabsorber, ein Vitamin und/oder ein ätherisches Öl enthalten ist oder zugesetzt wird.

6. Verfahren nach einem der vorhergehenden Ansprüche, wobei der Anteil des Wirkstoffs in dem in Schritt B) erhaltenen Gemisch ≤ 5 Gew.-%, insbesondere ≤ 1 Gew.-% beträgt.

7. Verfahren nach einem der vorhergehenden Ansprüche, wobei in Schritt B) zusätzlich Hilfsstoffe, insbesondere Konservierungsmittel zugemischt werden.

8. Wirkstoffhaltiger Schaum herstellbar mit dem Verfahren nach einem der Ansprüche 1 bis 7.

9. Schaum nach dem vorhergehenden Anspruch, wobei der Anteil nicht gasförmiger Bestandteile 20-35 Vol% beträgt.

## Claims

1. Process for producing an active ingredient-containing foam comprising a protein and also a fat and/or a wax, comprising the following steps:
A) providing the fat and/or the wax (fatty acid ester component) and the protein component, wherein
- the fatty acid ester component and/or the protein component comprise a foam-stabilizing constituent which comprises or consists of a foam-stabilizing protein present in the protein component and/or a foam-stabilizing constituent present in the fatty acid ester component,
- the protein component and the fatty acid ester component are present in a ratio by weight from 1:10 to 10:1,
- the fatty acid ester component consists of a plant oil or a plant wax
- wherein the protein component comprises or consists of a plant protein, especially a cereal protein, a pulse protein or a potato protein, or an egg protein or a milk protein,
- at least one pharmaceutical and/or cosmetic active ingredient has been introduced, is introduced or can be introduced in step C) into the fatty acid ester component,
B) mixing the protein component and the fatty acid ester component with addition of a thickener and with addition of water, so that water and the total proportions of protein component and fatty acid ester component are present in a ratio by weight from 1:1 to 20:1 and wherein the proportion of fatty acid ester component in the resulting mixture is greater than 3% by weight,
wherein no synthetic or partially synthetic surfactants are admixed,
C) introducing a fat-soluble gas selected from nitrogen, nitrous oxide, xenon and mixtures thereof into the mixture obtained according to step B) such that a foam is formed.

2. Process according to any of the preceding claims, wherein the fatty acid ester component is an oil or a wax that is liquid at 37°C.

3. Process according to either of the preceding claims, wherein the proportion of the fatty acid ester component in the mixture obtained in step B) is 5 to 50% by weight, and especially between 10 and 35% by weight.

4. Process according to any of the preceding claims, wherein at least 90% by weight of the proteins present in the protein component have a molecular weight ≤ 10 kDa.

5. Process according to any of the preceding claims, wherein as active ingredient at least a drug, an antibacterial substance, an antimicrobial substance, an antiperspirant, an antioxidant, a care substance, an odour absorber, a vitamin and/or an essential oil is present or is added.

6. Process according to any of the preceding claims, wherein the proportion of active ingredient in the mixture obtained in step B) is ≤ 5% by weight, especially ≤ 1% by weight.

7. Process according to any of the preceding claims, wherein additional auxiliaries, especially preservatives, are admixed in step B).

8. Active ingredient-containing foam producible by the process according to any of Claims 1 to 7.

9. Foam according to the preceding claim, wherein the proportion of non-gaseous constituents is 20-35% by volume.

## Revendications

1. Procédé de fabrication d'une mousse contenant un agent actif, qui comprend une protéine, ainsi qu'une graisse et/ou une cire, comprenant les étapes suivantes :
A) la mise à disposition de la graisse et/ou de la cire (composant ester d'acide gras) et du composant protéine,
- le composant ester d'acide gras et/ou le composant protéine contenant un constituant stabilisant la mousse, qui comprend une protéine stabilisant la mousse contenue dans le composant protéine et/ou un constituant stabilisant la mousse contenu dans le composant ester d'acide gras ou en est constitué,
- le composant protéine et le composant ester d'acide gras étant présents en un rapport en poids de 1:10 à 10:1,
- le composant ester d'acide gras étant constitué par une huile végétale ou une cire végétale,
- le composant protéine comprenant une protéine végétale, notamment une protéine céréalière, une protéine légumineuse ou une protéine de pomme de terre, ou une protéine d'œuf ou une protéine de lait ou en étant constitué,
- au moins un agent actif pharmaceutique et/ou cosmétique étant déjà introduit, étant ensuite introduit ou pouvant être introduit dans l'étape C) dans le composant ester d'acide gras,
B) le mélange du composant protéine et du composant ester d'acide gras avec ajout d'un épaississant et avec ajout d'eau, de telle sorte que l'eau et les proportions totales de composant protéine et de composant ester d'acide gras soient présentes en un rapport en poids de 1:1 à 20:1, et la proportion du composant ester d'acide gras dans le mélange formé étant supérieure à 3 % en poids,
aucun tensioactif synthétique ou partiellement synthétique n'étant incorporé,
C) l'introduction d'un gaz soluble dans les graisses, qui est choisi parmi l'azote, le gaz hilarant, le xénon et les mélanges de ceux-ci dans le mélange obtenu après l'étape B), de telle sorte qu'une mousse se forme.

2. Procédé selon l'une quelconque des revendications précédentes, dans lequel le composant ester d'acide gras est une huile ou une cire, qui est liquide à 37 °C.

3. Procédé selon l'une quelconque des revendications précédentes, dans lequel la proportion du composant ester d'acide gras dans le mélange obtenu dans l'étape B) est de 5 à 50 % en poids, et est notamment comprise entre 10 et 35 % en poids.

4. Procédé selon l'une quelconque des revendications précédentes, dans lequel au moins 90 % en poids des protéines contenues dans le composant protéine présentent un poids moléculaire ≤ 10 kDa.

5. Procédé selon l'une quelconque des revendications précédentes, dans lequel au moins un médicament, une substance antibactérienne, une substance antimicrobienne, un antitranspirant, un antioxydant, une substance de soin, un absorbeur d'odeur, une vitamine et/ou une huile éthérée sont contenus ou sont ajoutés en tant qu'agent actif.

6. Procédé selon l'une quelconque des revendications précédentes, dans lequel la proportion de l'agent actif dans le mélange obtenu dans l'étape B) est ≤ 5 % en poids, notamment ≤ 1 % en poids.

7. Procédé selon l'une quelconque des revendications précédentes, dans lequel des adjuvants, notamment des conservateurs, sont en outre incorporés dans l'étape B) .

8. Mousse contenant un agent actif pouvant être fabriquée avec le procédé selon l'une quelconque des revendications 1 à 7.

9. Mousse selon la revendication précédente, dans laquelle la proportion de constituants non gazeux est de 20 à 35 % en volume.
